# EUROPEAN PATENT SPECIFICATION

(11) **EP 0 614 972 B1**
(45) Date of publication and mention of the grant of the patent: **03.09.2003**
(21) Application number: 94301768.1
(22) Date of filing: 11.03.1994
(51) Int. Cl.: C12N 9/80, C12P 19/26

(54) **N-Acetyl-D-glucosamine deacetylase and a process for preparing the same**
N-Acetyl-D-Glucosamin Deacetylase und Verfahren zu deren Herstellung
N-acétyl-D-glucosamine déacétylase et procédé pour sa préparation

(30) Priority: 12.03.1993 JP 7865393
(43) Date of publication of application: 14.09.1994
(73) Proprietor: AGENCY OF INDUSTRIAL SCIENCE AND TECHNOLOGY, Chiyoda-ku Tokyo (JP)
(72) Inventor: Yamano, Naoko, Osaka-shi, Osaka-fu (JP); Tanaka, Ryutarou, Osaka-shi, Osaka-fu (JP); Fujishima, Shizu, Ikeda-shi, Osaka-fu (JP)
(74) Representative: Sturt, Clifford Mark

(56) References cited:
- DATABASE WPI Week 9303 Derwent Publications Ltd., London, GB; AN 93-022707 & JP-A-04 349 884 (AGENCY OF IND. SCI. & TECHNOLOGY) , 4 December 1992 & US-A-5 252 468 (FUJISHIMA ET AL.)
- BIOSCI. BIOTECH. BIOCHEM., vol. 58, no. 1, January 1994 pages 193-195, YAMANO N. ET AL. 'Production of N-acetylglucosamine deacetylase by Vibrio cholerae non-O1'

## Description

### BACKGROUND OF THE INVENTION

### Field of the Invention

The present invention relates to an enzyme N-acetyl-D-glucosamine deacetylase capable of hydrolyzing the acetamide group of N-acetyl-D-glucosamine to produce D-glucosamine and acetic acid which acts specifically on the monomer of N-acetyl-D-glucosamine, and not on the oligomers including dimer and the polymer.

The present invention also relates to a process for preparing the above described enzyme, and to a process for hydrolyzing said acetamido group using the deacetylase of the present invention.

### Description of the Prior Art

D-glucosamine, the deacetylated product of N-acetyl-D-glucosamine, is useful as pharmaceuticals, foodstuffs, and intermediates for the preparation of pharmaceuticals. In addition, oligosaccharides consisting of D-glucosamine unit have increasingly been drawing attention and becoming important as having valuable physiological activities such as antibacterial, antifungal and antitumor activities.

D-glucosamine has hitherto been prepared from N-acetyl-D-glucosamine which is prepared from purified chitin (β-1,4-poly-N-acetyl-D-glucosamine, obtained from the cuticle (crust) of a crustacean such as prawn or crab by removing inorganic salts, proteins and lipids), by deacetylating with a conc. alkali or mineral acid.

However, the hydrolysis of the N-acetyl group of N-acetyl-D-glucosamine does not progress so easily and therefore, relatively hard reaction conditions are necessary to complete the hydrolysis, for example, heating for a long period of time in a concentrated acid or alkali such as 30-60 % solution, leading to an increase in the formation of undesirable by-products, and the yield of the desired D-glucosamine is low. Moreover, the elimination of the acid or alkali used necessitates additional elaboration and expense.

There has been a need for developing more mild methods of deacetylating N-acetyl-D-glucosamine in a high yield without the formation of undesirable by-products, and a biological method using an enzyme capable of deacetylating N-acetyl-D-glucosamine has been investigated.

As biological deacetylation methods, there have been several references known wherein microorganisms belonging to Mucor sp., Aeromonas sp. or Colletotrium sp. are used.

In a method using a microorganism belonging to Mucor sp., soluble glycol chitin and the pentamer are deacetylated to the extent of 12-14 %, but there is no disclosure of a deacetylase acting only on the monomer of N-acetyl-D-glucosamine [Y. Araki, E. Ito, Biochem. Biophys. Res. Commun., 56: 669 (1974); Eur. J. Biochem., 55: 71 (1975); Methods Enzymol., 161: 510 (1988); L. L. Davis, S. Bartnicki-Garcia, Biochemistry, 23: 1065 (1984); C. Calvo-Mendez, J. Ruiz-Herrera, Exp. Mycol., 11: 128 (1987)].

A method using a microorganism belonging to Aeromonas sp. intends to deacetylate solid chitin of high polymerization degree, and there is no disclosure of deacetylating monosaccharide [K. Shimahara, H. Iwasaki, Asahi Garasu Kogyo Gijutsu Shoreikai, 41: 299 (1982), C.A. 99:84876v (1983)].

There is no disclosure also of deacetylating monosaccharide using a microorganism belonging to Colletotrium sp. [H. Kauss, W. Jeblick, D. H. Young, Plant Sci. Lett. 28: 231 (1983); H. Kauss, B. Bauch, Methods in Enzymology, 161: 518 (1988)]. Database WPI Week 9303 Derwent Publications AN 93-022707 (Agency of Ind. Sci. & Technology), 4th December 1992 discloses the finding of N-acetyl-D-glucosamine deacetylase activity towards monomeric and oligomeric forms of N-acetyl-D-glucosamine in Vibrio sp. cell extracts.

It has now surprisingly been found that a marine microorganism belonging to Vibrio sp. produces an enzyme which is capable of splitting the acetyl group of N-acetyl-D-glucosamine and which specifically acts on the monomer of N-acetyl-D-glucosamine and not the oligomer or polymer thereof.

The enzyme as described above having such novel substrate specificity has never been known hitherto, and represents a useful functional enzyme.

### SUMMARY OF THE INVENTION

Thus the present invention relates to a purified novel enzyme N-acetyl-D-glucosamine deacetylase obtainable from Vibrio sp. and capable of hydrolyzing acetamide group of N-acetyl-D-glucosamine to produce D-glucosamine and acetic acid, which has the following physicochemical properties:
(1) Substrate specificity: N-acetyl-D-glucosamine monomer, not the oligomer or polymer thereof;
(2) Optimal pH: 7.8 - 8.2 at 37°C;
(3) Stable pH: 6.0 - 9.0 at 45°C;
(4) Optimal temperature: 28 - 39°C;
(5) Molecular weight: 80,000-150,000
wherein said N-acetyl-D-glucosamine deacetylase is isolated by means of a purification process.

Furthermore, the present invention relates to the said enzyme wherein the Vibrio sp. is Vibrio cholerae non-01 (IFO 15429).

The present invention also relates to a process for preparing N-acetyl-D-glucosamine deacetylase as mentioned above, by incubating a microorganism belonging to Vibrio sp. capable of producing said deacetylase in an induction medium, and isolating said deacetylase from cells separated from said medium.

This invention further relates to a process as described above wherein the microorganism is previously incubated in a nutrition medium.

This invention also relates to a process as described above wherein the microorganism is Vibrio cholerae non-01 (IFO 15429).

The present invention also relates to a process for hydrolyzing acetamido group of N-acetyl-D-glucosamine monomer using the deacetylase of the present invention.

### Brief Description of the Drawings

Fig. 1 shows the relationship between the relative activity (%) of N-acetyl-D-glucosamine deacetylase of the present invention and the pH value, at 37 °C.
Fig. 2 shows the relationship between the relative activity (%) of N-acetyl-D-glucosamine deacetylase of the present invention and the temperature (°C), at pH 7.8.
Fig. 3 shows the relationship between the relative activity (%) of the N-acetyl-D-glucosamine deacetylase of the present invention and the pH value, at 37°C, after incubation at 45°C for a period of 30 minutes.
Fig. 4 shows the relationship between the relative activity (%) of the N-acetyl-D-glucosamine deacetylase of the present invention and the temperature (°C), after incubation at pH 7.8 at a given temperature for a period of 30 minutes.

### Preferred Embodiment of the Invention

The physicochemical properties of the enzyme of the present invention are described in detail hereinafter.

### 1) Action and substrate specificity:

The enzyme acts on the acetamide group of N-acetyl-D-glucosamine and hydrolyzes the latter to D-glucosamine and acetic acid, and substantially does not affect the oligomers, including dimers, of N-acetyl-D-glucosamine.

### 2) Optimal pH and stable pH:

The optimal pH of the enzyme of the present invention varies widely depending on the origin of the microorganism used to produce the enzyme, but is usually in the range of about 4.0 - 9.0. For example, the optimal pH of the enzyme derived from Vibrio sp. is in the range of 7.8 - 8.2 as determined colorimetrically according to the indole-HCl method at pH 5.6-10.8. The buffer solution used at pH 5.6-6.0 is 100mM CH₃COOH-CH₃COONa. At pH 6.0-9.0, 100mM KH₂HPO₄-Na₂HPO₄ is used, and at pH 9.6-10.8, 200mM Na₂HPO₄-NaOH is used.

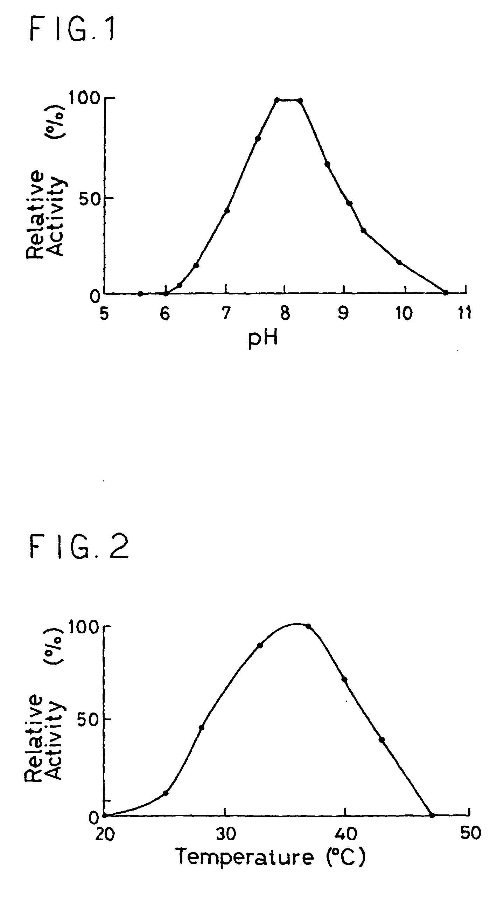

The relative activity of the enzyme obtained from Vibrio sp. is shown in Fig. 1.

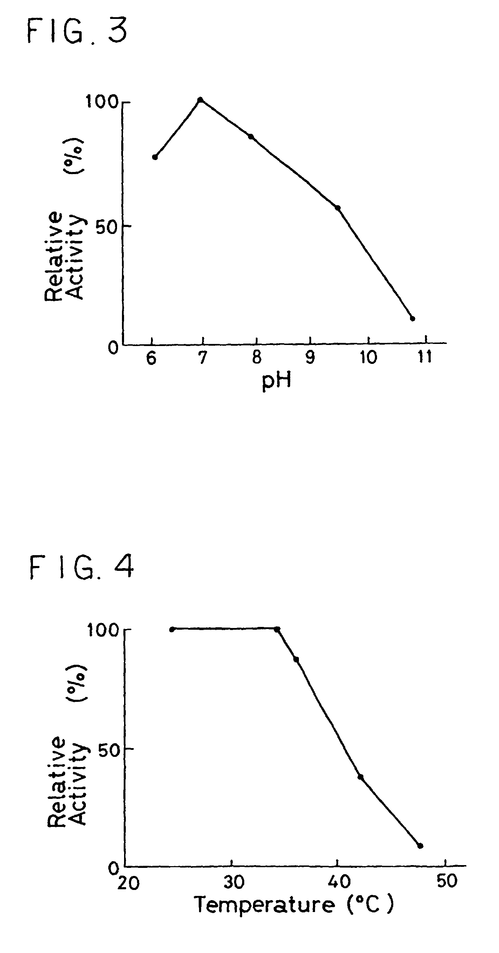

The stable pH wherein not less than 50% of the initial activity of the enzyme is retained, as determined at 37 °C after incubation in a given buffer at 45 °C for 30 minutes, varies depending on the origin of the microorganism used to produce the enzyme, but usually ranges from about 3.0 to 9.0. For example, the stable pH of the enzyme derived from Vibrio sp. is in the range of about 6.0-9.0. The relative activity after such incubation of the enzyme obtained from Vibrio sp. is shown in Fig. 3.

### 3) Optimal temperature range:

The optimal temperature for the enzymatic activity varies widely depending on the origin of the microorganism used to produce the enzyme, but usually ranges from about 10 °C to 50 °C. For example, the optimal temperature of the enzyme obtained from Vibrio sp. as determined at 20-47 °C is in the range of about 28-39 °C. The relative activity of the enzyme derived from Vibrio sp. is shown in Fig. 2.

### 4) Molecular weight:

The molecular weight of the enzyme as determined by TSK-gel G-3000 SW (RTM) column is 80,000 - 150,000.

N-acetyl-D-glucosamine deacetylase of the present invention can be obtained by incubating a microorganism belonging to Vibrio sp. Namely, a microorganism belonging to Vibrio sp. is incubated in a medium containing an inducer. Suitable inducers include chitin, chitin hydrolysate, N-acetyl-D-glucosamine and N-acetyl-D-glucosamine oligomers. These inducers can be used alone or in combination. Inducers are used in a concentration of about 0.1g/L or above, preferably at 1.0 - 50g/L.

The present invention is further described in detail referring to the production of the enzyme from a microorganism Vibrio cholerae non-01 (IFO 15429). Vibrio sp. microorganisms are publicly known. Among them, Vibrio cholerae non-01 (IFO 15429) was deposited at the INSTITUTE FOR FERMENTATION, OSAKA (IFO), 17-85, Jusohonmachi 2-chome, Yodogawa-ku, Osaka 532, Japan, under the accession number IFO 15429. The bacteriological properties of Vibrio cholerae non-01 (IFO 15429) are shown in Can. J Microbiol., Vol. 31, 711-720 (1985) and Bergey's Manual of Systematic Bacteriology Vol. 1, 518 (1957). In Bergey's Manual of Systematic Bacteriology, Vibrio Pacini corresponds to Vibrio cholerae non-01.

### 1) Incubation conditions:

The cells of a Vibrio cholerae non-01 (IFO 15429) microorganism are at first incubated in a conventional nutrition medium which need not be specifically defined. For example, such a conventional medium contains glucose, maltose, xylose, sucrose etc as a carbon source; yeast extract, peptone, meat extract, organic nitrogen sources such as aminoacid, inorganic nitrogen sources such as ammonium sulfate, ammonium chloride etc as a nitrogen source; and magnesium sulfate, magnesium chloride, sodium phosphate, potassium phosphate, potassium chloride, sodium chloride, calcium chloride etc as a mineral source, in appropriate combinations. The pH of the medium is adjusted between 6.5 - 8.0 by the addition of an appropriate acid or base, and the medium is autoclaved. Incubation is conducted aerobically at 25 - 40°C, preferably at 37°C, for 5 - 24 hours with stirring or agitation. The cells from the culture as incubated above are used for the production of N-acetyl-D-glucosamine deacetylase of the present invention by incubating said cells in an induction medium containing an inducer in addition to a carbon source, a nitrogen source and a mineral source.

For example, an induction medium contains 3g of ammonium nitrate, 1g of dipotassium hydrogen phosphate, 20g of sodium choloride. 0.5g of magnesium sulfate, 0.08g of calcium chloride, 50g of N-acetyl-D-glucosamine in 1L of the medium of pH 7.4.

Vibrio cholerae non-01 (IFO 15429) is aerobically incubated at 25-40 °C, preferably at 37 °C for 1-3 days while stirring or agitating.

### (2) Isolation of the enzyme:

The enzyme of the present invention can be recovered and purified from the culture using one or more conventional methods. For example, the culture thus incubated is centrifuged to collect cells, and the cells are disrupted by sonication, to give cell extract. The extract is purifed using ion exchange chromatography, adsorption chromatography, gel filtration chromatography, etc. A purification procedure comprises, for example, the following steps:
(1) the cells are disrupted by the treatment with supersonic waves and centrifuged to give cell-free extract;
(2) The cell-free extract is subjected to DEAE Bio-Gel chromatography and eluted with 10mM phosphate buffer containing 150mM NaCl;
(3) subjected further to hydroxyapatite column chromatography and eluted with 50mM phosphate buffer; and
(4) subjected to HPLC using TSK-gel 3000 SW gel filtration column.

The enrichment degree of the enzyme at each purification step is shown in Table 1 below, wherein the activity is determined by the method as described hereinafter.

**Table 1**

| Puri. Step | Tot.Prot. mg | Tot.Act. Unit | Spec.Act. Unit/mg | Enrich. Degree | Recov. % |
|---|---|---|---|---|---|
| (1) | 1730 | 6.20 | 0.00358 | 1 | 100 |
| (2) | 68.6 | 6.00 | 0.0875 | 24.4 | 96.7 |
| (3) | 6.57 | 2.45 | 0.373 | 104 | 39.5 |
| (4) | 0.72 | 0.88 | 1.22 | 346 | 14.2 |

As evident from Table 1, after the purification step (4), an enzyme having specific activity of 1.22 unit/mg protein (enriched 346-fold the activity of the initial value) can be obtained. Using this enzyme, enzymatic characteristics as described hereinafter are investigated.

### (3) The determination of the enzymatic activity:

An enzyme solution (0.1ml) is added to 0.3 ml of 100mM phosphate buffer (pH 7.8) containing 0.1ml of 1% N-acetyl-D-glucosamine as a substrate, and the resultant mixture is incubated at 37 °C for 30 minutes. The amount of D-glucosamine formed is determined colorimetrically using indole-HCl according to the method of Z. Dische and E. Borenfreund [J. Biol. Chem., 184: 517 (1950)]. One unit of the enzyme is defined as the amount of the enzyme which is capable of producing 1 µmol D-glucosamine from N-acetyl-D-glucosamine per minute at 37 C.

As described above, N-acetyl-D-glucosamine deacetylase of the present invention is a novel enzyme which has not been known hitherto and which enables deacetylation of monomeric N-acetyl-D-glucosamine in a mild condition to produce D-glucosamine. It is possible from the substrate specificity of this enzyme to deacetylate only monomeric N-acetyl-D-glucosamine, among the mixture thereof with oligomers. The deacetylated product D-glucosamine is useful as a starting material for the preparation of many pharmaceuticals, functional oligosaccharides, etc., and has a wide range of utility.

The present invention is further illustrated by the following EXAMPLE.

### EXAMPLE

### 1. Incubation of Vibrio cholerae non-01 strain (IFO 15429)

Vibrio cholerae non-01 strain (IFO 15429) was incubated in a plate agar medium containing 0.6g of ammonium nitrate, 0.2g of dipotassium hydrogen phosphate, 2g of sodium chloride, 0.12g of magnesium sulfate, 0.02g of calcium chloride, 10g of glucose and 3g of agar per 200ml of the medium of pH 7.4 at 37 C for 24 hours, and the cells were collected using a spatula. The cells thus obtained were then aerobically incubated with shaking for 24 hours in the following induction medium. Induction medium: 1.5g of ammonium nitrate, 0.5g of dipotassium hydrogen phosphate, 10g of sodium chloride, 0.3g of magnesium sulfate, 0.04g of calcium chloride, and 25g of N-acetyl-D-glucosamine in 500ml of a liquid medium of pH 7.4.

### 2. Recovery and purification of the enzyme

The culture thus obtained was cetrifuged at 8000 g for 20 minutes to afford 11g of wet cells. The cells were suspended in 40ml of physiological saline and sonicated at 0 C for 10 minutes (20 seconds operations at intervals of 20 seconds), then centrifuged to give cell-free extract containing 1.73g of a protein having specific activity of 0.00358 unit/mg protein.

The cell-free extract as obtained above was applied to a column charged with DEAE Bio-Gel A which had been equilibrated with 0.01M phosphate buffer (pH 7.0), and eluted with a stepwise NaCl gradient eluent. Using 150mM NaCl, 68.6mg of a protein having specific activity of 0.0875 U/mg protein was eluted. The eluate was further applied to a hydroxyapatite column and eluted with a stepwise gradient phosphate buffer. 10ml of an eluate containing 2.45mg of a protein having specific activity of 0.373 U/mg protein was eluted using 50mM phosphate buffer.

The eluate (10ml) thus obtained was concentrated to 0.4ml by ultrafiltration (exclusion limit 100,000) and subjected to HPLC using TSK gel 3000 SW column to give 0.72 mg of purified enzyme having specific activity of 1.22 U/mg protein. The enrichment degree of the activity was 346-fold of the initial value and the recovery of the enzyme was 14.2%. The molecular weight of this enzyme was 80,000-150,000.

### 3. Substrate specificity of the enzyme

The substrate specificity of the enzyme as obtained above was investigated using monomer and several oligomers (2-6 mers) of N-acetyl-D-glucosamine (SEIKAGAKU KOGYO CO. LTD. Japan), and chitin as a substrate. These substrates were added at a concentration of 0.2%, and the enzymatic reaction was allowed to take place at a temperature of 37 °C and a pH of 7.8 for one hour. The amount of D-glucosamine formed was determined using indole-HCl according to the method of Z. Dische and E. Borenfreund [J. Biol. Chem., 184: 517 (1950)]. The results obtained are shown in Table 2 below.

**Table 2**

| Substrate | Enzymatic Activity Unit/mg | Relative Activity % |
|---|---|---|
| GlcNAc | 1.22 | 100 |
| (GlcNAc)₂ | Not detected | - |
| (GlcNAc)₃ | Not detected | - |
| (GlcNAc)₄ | Not detected | - |
| (GlcNAc)₅ | Not detected | - |
| (GlcNAc)₆ | Not detected | - |
| Chitin | Not detected | - |
| One unit of enzyme activity is defined as the amount of the enzyme required to form 1µ equivalent of the amino group per min at 37°C. | | |

As seen from Table 2 above, formation of D-glucosamine from monomeric N-acetyl-D-glucosamine can be observed while none of the oligomers tested (2-mer to 6-mer) and chitin show the formation of D-glucosamine.

## Claims

1. A purified N-acetyl-D-glucosamine deacetylase obtainable from Vibrio sp. and capable of hydrolyzing acetamide group of N-acetyl-D-glucosamine to produce D-glucosamine and acetic acid, which has the following physicochemical properties:
(1) Substrate specificity: N-acetyl-D-glucosamine monomer, not the oligomer or polymer thereof;
(2) Optimal pH: 7.8 - 8.2 at 37°C;
(3) Stable pH: 6.0 - 9.0 at 45°C;
(4) Optimal temperature: 28 - 39°C;
(5) Molecular weight: 80,000-150,000
wherein said N-acetyl-D-glucosamine deacetylase is isolated by means of a purification process.

2. A purified N-acetyl-D-glucosamine deacetylase according to claim 1, wherein said purification process comprises any one of ion exchange chromatography, adsorption chromatography or gel filtration chromatography.

3. A purified N-acetyl-D-glucosamine deacetylase according to claim 1, wherein said purification process comprises subjecting said deacetylase to:
chromatography and elution with 10mM phosphate buffer containing 150mM NaCl;
hydroxyapatite column chromatography and elution with 50mM phosphate buffer; and
HPLC using a gel filtration column.

4. A purified N-acetyl-D-glucosamine deacetylase as claimed in claim 1, wherein the Vibrio sp. is Vibrio cholerae non-01 (IFO 15429).

5. A process for preparing N-acetyl-D-glucosamine deacetylase capable of hydrolyzing the acetamide group of N-acetyl-D-glucosamine to produce D-glucosamine and acetic acid and having the following physicochemical properties:
(1) Substrate specificity: N-acetyl-D-glucosamine monomer, not the oligomer or polymer thereof;
(2) Optimal pH: 7.8 - 8.2 at 37°C;
(3) Stable pH: 6.0 - 9.0 at 45°C;
(4) Optimal temperature: 28 - 39°C;
(5) Molecular weight: 80,000-150,000 which comprises incubating a microorganism belonging to Vibrio sp. and capable of producing said deacetylase in an induction medium, and isolating said deacetylase from cells separated from said medium.

6. A process as claimed in claim 5, wherein the microorganism is previously incubated in a nutrition medium.

7. A process as claimed in claim 6, wherein the microorganism is Vibrio cholerae non-01 (IFO 15429).

8. A process for hydrolyzing acetamido group of N-acetyl-D-glucosamine monomer which comprises conducting the hydrolysis using N-acetyl-D-glucosamine deacetylase as claimed in claim 1.

## Patentansprüche

1. Gereinigte N-Acetyl-D-glucosamin-deacetylase, erhältlich aus Vibrio sp. und in der Lage, die Acetamidgruppe von N-Acetyl-D-glucosamin unter Erzeugung von D-Glucosamin und Essigsäure zu hydrolysieren und welche die folgenden physiko-chemischen Eigenschaften aufweist:
(1) Substratspezifität: N-Acetyl-D-glucosamin-Monomer, nicht das Oligomer oder Polymer davon;
(2) Optimaler pH-Wert: 7,8-8,2 bei 37°C;
(3) Stabiler pH-Wert: 6,0-9,0 bei 45°C;
(4) Optimale Temperatur: 28-39°C;
(5) Molekulargewicht: 80.000-150.000,
wobei die N-Acetyl-D-glucosamin-deacetylase mittels eines Reinigungsverfahrens isoliert wurde.

2. Gereinigte N-Acetyl-D-glucosamin-deacetylase nach Anspruch 1, wobei das Reinigungsverfahren lonenaustauschchromatographie, Adsorptionschromatographie oder Gelfiltrationschromatographie umfaßt.

3. Gereinigte N-Acetyl-D-glucosamin-deacetylase nach Anspruch 1, wobei das Reinigungsverfahren das Unterziehen der Deacetylase von
Chromatographie und Elution mit 10 mM Phosphatpuffer, welcher 150 mM NaCI enthält,
Hydroxylapatit-Säuienchromatographie und Elution mit 50 mM Phosphatpuffer und
HPLC unter Verwendung einer Gelfiltrationssäule
umfaßt.

4. Gereinigte N-Acetyl-D-glucosamin-deacetylase nach Anspruch 1, wobei Vibrio sp. Vibrio cholerae non-01 (IFO 15429) ist.

5. Verfahren zur Herstellung von N-Acetyl-D-glucosamin-deacetylase, welche in der Lage ist, die Acetamidgruppe von N-Acetyl-D-glucosamin unter Erzeugung von D-Glucosamin und Essigsäure zu hydrolysieren, und welche die folgenden physiko-chemischen Eigenschaften aufweist:
(1) Substratspezifität: N-Acetyl-D-glucosamin-Monomer, nicht das Oligomer oder Polymer davon;
(2) Optimaler pH-Wert: 7,8-8,2 bei 37°C;
(3) Stabiler pH-Wert: 6,0-9,0 bei 45°C;
(4) Optimale Temperatur: 28-39°C;
(5) Molekulargewicht: 80.000-150.000,
welches Inkubieren eines Mikroorganismus, der zu Vibrio sp. gehört und in der Lage ist, die Deacetylase herzustellen, in einem Induktionsmedium und Isolieren der Deacetylase aus Zellen, die von dem Medium getrennt sind, umfaßt.

6. Verfahren nach Anspruch 5, wobei der Mikroorganismus zuvor in einem Nährmedium inkubiert wird.

7. Verfahren nach Anspruch 6, wobei der Mikroorganismus Vibrio cholerae non-01 (IFO 15429) ist.

8. Verfahren zum Hydrolysieren der Acetamido-Gruppe von N-Acetyl-D-glucosamin-Monomer, welches die Durchführung der Hydrolyse unter Verwendung von N-Acetyl-D-glucosamin-deacetylase nach Anspruch 1 umfaßt.

## Revendications

1. N-acétyl-D-glucosamine-désacétylase purifiée pouvant être obtenue à partir de Vibrio sp. et capable d'hydrolyser le groupe acétamide de la N-acétyl-D-glucosamine pour produire de la D-glucosamine et de l'acide acétique, qui présente les propriétés physico-chimiques suivantes :
(1) spécificité du substrat : monomère de N-acétyl-D-glucosamine, pas l'oligomère ni le polymère de celle-ci ;
(2) pH optimal : 7,8-8,2 à 37°C ;
(3) pH stable : 6,0-9,0 à 45°C ;
(4) température optimale : 28-39°C ;
(5) poids moléculaire : 80000-150000 ;
laquelle N-acétyl-D-glucosamine-désacétylase est isolée au moyen d'un procédé de purification.

2. N-acétyl-D-glucosamine-désacétylase purifiée selon la revendication 1, dans laquelle ledit procédé de purification comprend l'une quelconque d'une chromatographie par échange ionique, d'une chromatographie par adsorption ou d'une chromatographie par filtration sur gel.

3. N-acétyl-D-glucosamine-désacétylase purifiée selon la revendication 1, dans laquelle le procédé de purification comprend la soumission de ladite désacétylase à :
une chromatographie et une élution avec un tampon phosphate à 10 mM contenant 150 mM de NaCl,
une chromatographie sur colonne d'hydroxyapatite et une élution avec un tampon phosphate à 50 mM et
une HPLC en utilisant une colonne de filtration sur gel.

4. N-acétyl-D-glucosamine-désacétylase purifiée telle que revendiquée dans la revendication 1, dans laquelle la Vibrio sp. est la Vibrio cholerae non-01 (IFO 15429).

5. Procédé pour préparer de la N-acétyl-D-glucosamine-désacétylase capable d'hydrolyser le groupe acétamide de la N-acétyl-D-glucosamine pour produire de la D-glucosamine et de l'acide acétique et présentant les propriétés physico-chimiques suivantes :
(1) spécificité du substrat : monomère de N-acétyl-D-glucosamine, pas l'oligomère ni le polymère de celle-ci ;
(2) pH optimal : 7,8-8,2 à 37°C ;
(3) pH stable : 6,0-9,0 à 45°C ;
(4) température optimale : 28-39°C ;
(5) poids moléculaire : 80000-150000 ;
qui comprend l'incubation d'un micro-organisme appartenant à Vibrio sp. et capable de produire ladite désacétylase dans un milieu d'induction, et l'isolement de ladite désacétylase des cellules séparées dudit milieu.

6. Procédé tel que revendiqué dans la revendication 5, dans lequel le micro-organisme est incubé au préalable dans une colonne de nutrition.

7. Procédé tel que revendiqué dans la revendication 6, dans lequel le micro-organisme est la Vibrio cholerae non-01 (IFO 15429).

8. Procédé pour hydrolyser le groupe acétamide du monomère de la N-acétyl-D-glucosamine, qui comprend la réalisation de l'hydrolyse en utilisant une N-acétyl-D-glucosamine-désacétylase telle que revendiquée dans la revendication 1.
